# EUROPEAN PATENT APPLICATION

(11) **EP 4 706 528 A1**
(43) Date of publication of application: **11.03.2026**
(21) Application number: 24800229.7
(22) Date of filing: 02.05.2024
(51) Int. Cl.: A61B 5/145, A61B 90/00

(54) **INSERTION DEVICE ASSEMBLY FOR INSERTING ANALYTE MONITORING DEVICE**

(30) Priority: 02.05.2023 KR 20230057105
(71) Applicant: SD Biosensor, Inc., Gyeonggi-do 16690 (KR)
(72) Inventor: LEE, Hyo Keun, Suwon-si, Gyeonggi-do 16690 (KR); PARK, Hyo-Lim, Suwon-si, Gyeonggi-do 16684 (KR); HONG, Soonmin, Hwaseong-si, Gyeonggi-do 18477 (KR); JEON, Sangyeun, Yongin-si, Gyeonggi-do 17091 (KR)
(74) Representative: J A Kemp LLP
(86) International application number: PCT/KR2024/005930
(87) International publication number: WO 2024/228548

(57) **Abstract**

The present invention relates to an insertion device assembly for inserting a device for monitoring body analytes. The insertion device assembly can include: a transmitter including an upper case, a lower case, a sensor, a holder, and a through-hole, an applicator including an upper housing, a lower housing, a needle assembly, a needle carrier, and a driving portion, and a needle assembly including a needle, a grip portion, a needle injection portion, and a protrusion.

## Description

### [Technical Field]

The present disclosure relates to an insertion device assembly, and more particularly, to an insertion device assembly for inserting a device for monitoring body analytes

### [Background Art]

Contents described in this section merely provide background information for the present disclosure and do not constitute prior art.

With the recent rise of preferences for processed foods, people are increasingly exposed to simple sugars. This environment has also led to a sharp rise in diabetes. Sudden drops or spikes in blood sugar can lead to shock in diabetic patients, and in rare cases, death. Thus, diabetic patients need to continuously monitor their blood sugar levels.

In blood sugar measurement devices according to the related art, a lancet is used to make an incision in the fingertip, and the diabetic patient's blood drawn from the incision is then inserted into a blood sugar analyzer to calculate the blood sugar level.

This type of blood sugar measurement device is painful and frightening to a user. Furthermore, it is unrealistic to make a finger bleed every hour.

To solve this problem, continuous glucose monitoring (CGM) systems have been developed. By attaching a microscopic sensor and an electronic device (hereinafter, referred to as a "transmitter") for analyzing the analyte (hereinafter, referred to as a "blood sugar") measured by the sensor to the skin of a diabetic patient, blood sugar levels are continuously measured for a period of a week to ten days.

A sensor of the transmitter is very thin, making it difficult to directly penetrate the skin. Thus, a configuration (hereinafter, referred to as an "applicator") is required to insert a needle into the skin, insert the sensor through the gap, and then withdraw the needle.

Meanwhile, according to US 2021-0038131 A1, the user has to insert a sensor introducer 106 into a sensor inserter 500. This requires aligning the sensor introducer's orientation and inserting the sensor introducer 106 into the sensor inserter, and in this procedure, there is a great concern that users will feel fatigued. Thus, it is preferable for the insertion device assembly to be manufactured with the applicator and transmitter integrated into one unit.

Furthermore, according to US 2022-0322975 A1, an on-skin assembly 102 is assembled within an applicator 100 without any sealing structure between the insertion assembly 118 and a sealing element 110, and thus, there is a risk of future exposure to contamination.

Furthermore, according to US 2021-0361197 A1, a needle unit 550 including a sensor unit 520 accommodated therein penetrates upper and lower parts of a body attachable unit 20, however, after the body attachable unit 20 is attached to the user's body and the needle unit 550 is removed, there is a risk of infection of the penetrated part in an open state due to entering of water or foreign substances.

Since a sensor probe 521 as one side of the sensor unit 520 that is exposed to the outside of the body attachable unit 20 and a sensor body unit 522 as the other side of the sensor unit 520 that is positioned within the body attachable unit 20 are bent at a right angle, there is a risk of damage to the sensor unit 520, which is formed of a flexible film.

In addition, sterilization of the applicator using sterilizing gas is time-consuming and carries the risk of residue. For this reason, E-beams are sometimes used for sterilizing medical devices, and in this case, there is a risk of affecting electronic devices.

Furthermore, it is difficult to inspect the interior of a typical applicator during the assembly process. This can lead to insufficient inspection of defective products.

Furthermore, it is significant to maintain sterile conditions after sterilization of the insertion device assembly. At this time, there is a problem that the manufacturing cost increases and waste increases due to double or triple packaging to maintain sterile conditions.

### [Disclosure]

### [Technical Problem]

Accordingly, the present disclosure provides an insertion device assembly in which an applicator and a transmitter are manufactured as one body and a user preparation operation can be simply performed.

In addition, the present disclosure also provides an insertion device assembly in which sterile conditions can be maintained by applying a double sealing structure between the applicator and the transmitter.

In addition, the present disclosure also provides an insertion device assembly in which there is no risk of malfunction even after E-beam type sterilization is performed.

In addition, the present disclosure also provides an insertion device assembly in which defects can be identified during the manufacturing process.

In addition, the present disclosure also provides an insertion device assembly that can be easily manufactured by simplifying a sealing operation for maintaining sterile conditions.

In addition, the present disclosure also provides an insertion device assembly in which, after a needle is removed from the transmitter, a part that the needle penetrates, can be sealed.

In addition, the present disclosure also provides an insertion device assembly that prevents damage of a sensor unit that is one of significant configurations being inserted into a user's body and for analyzing the concentration of body analytes.

The problems to be solved by the present invention are not limited to the problems mentioned above, and other problems not mentioned will be clearly understood by those skilled in the art from the description below.

### [Technical Solution]

According to an aspect of the present invention, there is provided an insertion device assembly including: a transmitter; and wherein the transmitter includes: an upper case; a lower case coupled to the upper case and forming an accommodation space therein; a sensor arranged on the lower case; a holder arranged inside the lower case and arranged on the sensor; and a lower through-hole, which is positioned in the lower case and in which the holder is positioned; an applicator on which the transmitter is mounted, wherein the applicator includes: an upper housing having an opening formed in a lower part of the upper housing and a button provided on an outer circumferential surface of the upper housing; a lower housing coupled to the opening of the upper housing, thereby forming an internal space with the upper housing; a needle assembly arranged at an upper part of the transmitter in the internal space and configured so that at least part of the needle assembly is inserted into the holder; a needle carrier arranged in the internal space and configured to allow the needle assembly to make linear motion; and a driving portion configured to provide power to the needle carrier, wherein the needle assembly includes: a needle configured to penetrate a user's skin; a grip portion coupled to one end of the needle carrier; a needle injection portion arranged between the needle and the grip portion and configured to be coupled to the holder; and
a protrusion protruding from an end of the needle injection portion and inserted into the holder.

According to another aspect of the present invention, there is provided an insertion device assembly including: a transmitter; and wherein the transmitter includes: an upper case; a lower case coupled to the upper case and forming an accommodation space therein; a sensor arranged on the lower case; a holder arranged inside the lower case and arranged on the sensor; and a lower through-hole, which is positioned in the lower case and in which the holder is positioned; and a packing portion positioned at the upper part of the holder and formed of an elastic material, an applicator on which the transmitter is mounted, wherein the applicator includes: an upper housing having an opening formed in a lower part of the upper housing and a button provided on an outer circumferential surface of the upper housing; a lower housing coupled to the opening of the upper housing, thereby forming an internal space with the upper housing; a needle assembly arranged at an upper part of the transmitter in the internal space and configured so that at least part of the needle assembly is inserted into the holder; a needle carrier arranged in the internal space and configured to allow the needle assembly to make linear motion; and a driving portion configured to provide power to the needle carrier, wherein the needle assembly includes: a needle configured to penetrate a user's skin; a grip portion coupled to one end of the needle carrier; and a needle injection portion arranged between the needle and the grip portion and configured to be coupled to the holder.

When the insertion device assembly is assembled, the protrusion may be inserted into the holder, and when the insertion device assembly is used, the needle injection portion may be withdrawn from the holder.

When the insertion device assembly is assembled, the needle may be positioned to penetrate the packing portion, and when the insertion device assembly is used, the needle may be removed from the holder, and a part of the packing portion that the needle penetrates may be closed by elasticity.

The upper part of the holder and an inner upper surface of the upper case may be integrally injection molded.

The insertion device assembly may further include a cap configured to be mounted on or removed from the upper housing, wherein the cap includes: an opening formed in a lower surface of the cap; a plurality of ribs of which at least part is arranged inside the lower housing and which extend radially inward from an inner circumferential surface of the cap; and a needle accommodation portion supported by the plurality of ribs and configured to surround at least part of the needle.

The needle assembly may further include a cap injection portion that corresponds to a shape of an upper surface of the needle injection portion and arranged on an upper surface of the needle accommodation portion, and the cap injection portion may come into contact with the lower through-hole of the lower case and seal the lower through-hole of the lower case.

The cap injection portion may include an elastic member.

The plurality of ribs may be two or more and arranged symmetrically around the needle accommodation portion, and the transmitter may further include an adhesive tape attached to a lower surface of the lower case and provided with a number of the plurality of ribs, and the adhesive tape may be inserted through the opening.

The transmitter may further include a printed circuit board (PCB) accommodated in an accommodation space of the upper case and the lower case and a dummy PCB connecting the sensor to the PCB.

The transmitter may further include an inclined surface formed to be inclined from one side of the lower through-hole downward, and the inclined surface may further include an inclined track on which part of the sensor is disposed.

The insertion portion of the sensor may be arranged inside the needle that penetrates the holder and is positioned, and two bent portions that form an obtuse angle may be formed between one end and the other end of the sensor by the inclined surface and the needle.

According to another aspect of the present invention, there is provided an insertion device assembly including: a transmitter; and an applicator configured in such a way that the transmitter is mounted on the applicator, wherein the insertion device assembly is divided into first through fourth assemblies depending on an assembly order, and the first assembly includes: a cap of which upper and lower parts are open; a lower housing arranged inside the cap; a lower case of the transmitter arranged on at least part of the lower housing; a sensor arranged on the lower case; a holder arranged inside the lower case and arranged on the sensor; and a needle assembly arranged at an upper part of the transmitter and configured in such a way that a protruding end of the needle assembly is inserted into the holder.

According to another aspect of the present invention, there is provided an insertion device assembly including: a transmitter; and an applicator configured in such a way that the transmitter is mounted on the applicator, wherein the insertion device assembly is divided into first through fourth assemblies depending on an assembly order, and the first assembly includes: a cap of which upper and lower parts are open; a lower housing arranged inside the cap; a lower case of the transmitter arranged on at least part of the lower housing; a sensor arranged on the lower case; a holder arranged inside the lower case and arranged on the sensor; a packing portion positioned at an upper part of the holder and formed of an elastic material; and a needle assembly, which is arranged at an upper part of the transmitter and in which a needle positioned at an end of the needle assembly is positioned to penetrate the packing portion.

To the first assembly sterilized using E-beam, the second assembly may further include: a PCB arranged on the lower case; and an upper case, which is coupled to the lower case and forms an accommodation space therein.

To the second assembly, the third assembly may further include: a needle carrier configured to allow the needle assembly to make linear motion; and a driving portion configured to provide power to the needle carrier.

To the third assembly, the fourth assembly may further include: an adhesive tap attached to a lower surface of the lower case; and a sealing portion configured to seal an open lower part of the cap.

The needle assembly may include: a needle configured to penetrate a user's skin; a grip portion coupled to one end of the needle carrier; a needle injection portion arranged between the needle and the grip portion and configured to be coupled to the holder; and a protrusion protruding from an end of the needle injection portion and inserted into the holder.

The cap may include: a plurality of ribs of which at least part is arranged inside the lower housing and which extend radially inward from an inner circumferential surface of the cap; and a needle accommodation portion supported by the plurality of ribs and configured to surround at least part of the needle; and a cap injection portion having a shape corresponding to a shape of an upper surface of the needle accommodation portion and arranged on the upper surface of the needle accommodation portion, and the cap injection portion may be positioned to contact a lower through-hole positioned in a lower surface of the lower case.

The plurality of ribs may be two or more and arranged symmetrically around the needle accommodation portion, and the transmitter may further include an adhesive tape attached to a lower surface of the lower case and provided with a number of the plurality of ribs, and the adhesive tape may be inserted through the open lower part of the cap.

### [Advantageous Effects]

As described above, according to an embodiment of the present disclosure, an applicator and a transmitter are manufactured as one body, and when the applicator is used, a needle protection configuration can be removed at once by simply removing a cap, so that convenience can be provided to a user.

In addition, a needle and the inside of the applicator can be maintained in sterile conditions using a double sealing structure of the insertion device assembly.

In addition, according to an embodiment of the present disclosure, when an insertion device assembly is assembled, a printed circuit board (PCB) is assembled after sterilization by E-beam is performed, so that there is no risk of malfunction of electronic devices mounted on the PCB.

In addition, a cap is formed of a transparent material so that, whether sealing is formed between a cap injection portion and the transmitter, can be confirmed with the naked eye and defective products can be easily inspected.

In addition, since, after the assembly of the insertion device assembly is completed, only a sealing portion has to be attached to a lower part of the cap, a sealing method is simple and thus, manufacturing can be conveniently performed.

In addition, a packing portion formed of an elastic material is positioned at a part where the needle penetrates and is arranged in the transmitter, so that a penetrated part is sealed after the needle is removed and entering of water or foreign substances and infection caused thereby can be prevented.

In addition, a sensor is installed using a track structure inside a case that constitutes the transmitter, so that a bent portion of the sensor is smoothly formed and thus damage to the sensor can be prevented.

### [Brief Description of the Drawings]

FIG. 1 is a front perspective view of an insertion device assembly according to an embodiment of the present disclosure.
FIG. 2 is an exploded perspective view of the insertion device assembly according to an embodiment of the present disclosure.
FIG. 3 is a perspective view and exploded perspective view of a transmitter according to an embodiment of the present disclosure.
FIG. 4 illustrates an inside of a cap according to an embodiment of the present disclosure.
FIG. 5 is a front perspective view of a lower housing according to an embodiment of the present disclosure.
FIG. 6 is a flowchart of an assembling method of the insertion device assembly according to an embodiment of the present disclosure.
FIG. 7 illustrates an assembly order of the insertion device assembly according to an embodiment of the present disclosure.
FIGS. 8 through 11 illustrate a state of an assembling operation of the insertion device assembly according to an embodiment of the present disclosure.
FIG. 12 is a perspective view of a connecting structure of a printed circuit board (PCB), a sensor, and a dummy PCB, which are positioned inside the transmitter, as viewed from below, according to an embodiment of the present disclosure,
FIG. 13 is an exploded perspective view of a connecting structure of a PCB, a sensor, and a dummy PCB, which are positioned inside the transmitter, according to an embodiment of the present disclosure.
FIGS. 14 and 15 are perspective views of a state where a track structure of a lower case and a sensor is installed at the lower case, according to an embodiment of the present disclosure.
FIGS. 16 and 17 are longitudinal cross-sectional views of the shape of a sensor in a state where a needle assembly is not coupled to the transmitter, and the shape of a sensor in a state where the needle assembly is coupled to the transmitter.
FIG. 18 illustrates a state where a holder and an upper case are formed as one unit, in the transmitter according to an embodiment of the present disclosure.
FIG. 19 illustrates coupling of the needle assembly and the transmitter according to another embodiment of the present disclosure.
FIG. 20 illustrates a state where the holder and the upper case are formed as one unit, in the transmitter according to another embodiment of the present disclosure.

### [Detailed Description of Preferred Embodiments]

Hereinafter, some embodiments of the present disclosure will be described in detail with reference to exemplary drawings. When adding reference numerals to components of each drawing, it should be noted that the same components are given the same numerals as much as possible even if they are shown in different drawings. In addition, when describing the present disclosure, if it is determined that a specific description of a related known configuration or function may obscure the gist of the present disclosure, the detailed description thereof will be omitted.

When describing components of embodiments according to the present disclosure, numerals such as first, second, i), ii), a), b, etc. may be used. These numerals are only for distinguishing the component from other components, and the nature, order, or sequence of the component is not limited by the numerals. When a part in the specification is said to "include" or "have" a component, this does not mean that other components may be excluded, but rather that other components may be included, unless explicitly stated otherwise.

In the present disclosure, an "insertion device assembly" represents an assembly in which an applicator and a transmitter are assembled.

In addition, in the present disclosure, a "distal position" refers to a position that is relatively farther from the skin than a "proximal position". Meanwhile, distal and proximal are concepts of relative position and can be understood that one element is both proximal and distal to another.

In addition, in the present disclosure, "fire" refers to a user's action of operating an applicator so that a needle is inserted into the user's skin.

In addition, in the present disclosure, "retraction" refers to a process by which the needle returns to its original position after being inserted into the user's skin.

In addition, in the present disclosure, "upward" refers to a direction from proximal to distal.

In addition, in the present disclosure, "downward" refers to a direction from distal to proximal.

In addition, in the present disclosure, "horizontal" refers to a plane that the applicator contacts when used, and may refer to a skin surface. In the present disclosure, the curvature of the skin surface is not considered, and an explanation assumes that the user's skin surface is flat.

### 1. Explanation of applicator

### 1.1 Structure of applicator

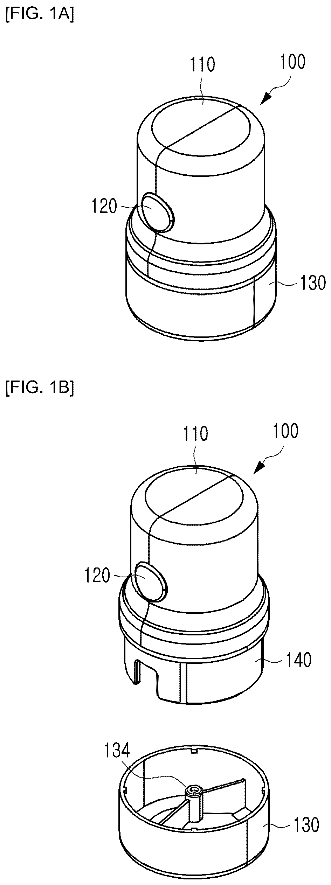

FIG. 1 is a front perspective view of an insertion device assembly according to an embodiment of the present disclosure.

Specifically, FIG. 1A illustrates a state in which a cap 130 is mounted, and FIG. 1B illustrates a state in which the cap 130 is removed.

An applicator 100 according to an embodiment of the present disclosure may include an upper housing 110, a button 120 configured to be inserted and pressed in an outer circumferential surface of the upper housing 110, and a cap 130 that can be removed from the upper housing 110 (see FIG. 1A).

When the cap 130 of the applicator 100 is removed from the upper housing 110, a portion of a lower housing 140 is exposed (see FIG. 1B). The cap 130 is removed so that the insertion device assembly becomes available for use by users.

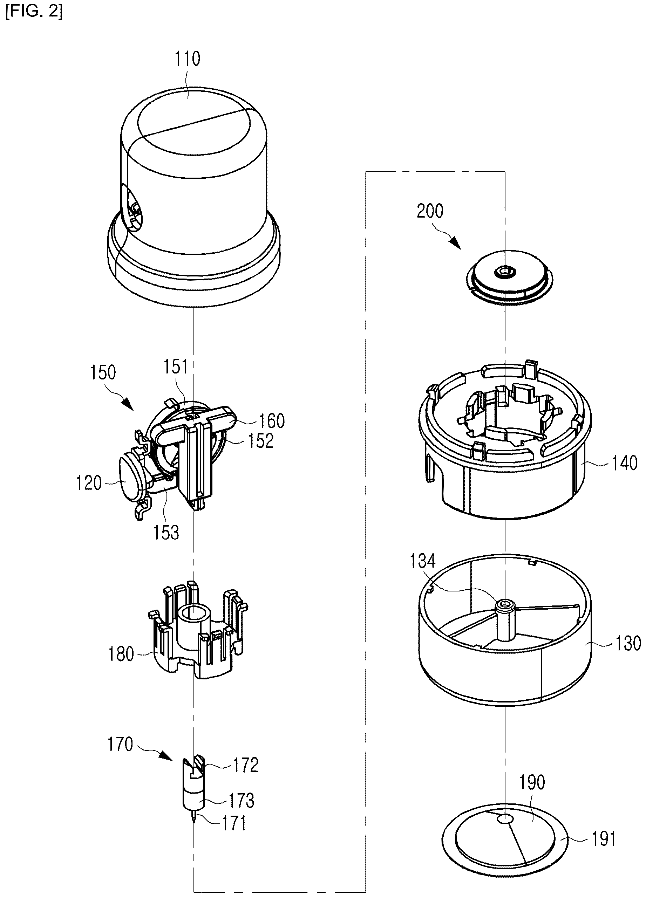

FIG. 2 is an exploded perspective view of an insertion device assembly according to an embodiment of the present disclosure.

Referring to FIG. 2, the applicator 100 according to an embodiment of the present disclosure may include all or part of a driving portion 150, a needle carrier 160, a needle assembly 170, a transmitter holder 180, a dehumidifier 190, and a sealing portion 191 in addition to the upper housing 110, the button 120, the cap 130, and the lower housing 140.

The upper housing 110 is configured to be gripped by a user and may have an approximately cylindrical shape. Meanwhile, the lower part of the upper housing 110 is open.

The button 120 is arranged and provided on an outer circumferential surface of the upper housing 110 and is configured to be pressed toward the inside of the upper housing 110.

The cap 130 is configured to be mounted or removed on or from the upper housing 110, and may protect the inside of the applicator 100 from external contaminants or shock. The cap 130 may have an approximately cylindrical shape.

The lower housing 140 is arranged inside the cap 130. The lower housing 140 is configured to be coupled to the lower part of the upper housing 110 and to form a space therein. When the applicator 100 is used, the lower housing 140 is configured to come into direct contact with the user's skin.

Referring further to FIG. 2, the driving portion 150 is arranged in an internal space formed by the upper housing 110 and the lower housing 140. The driving portion 150 is configured to allow the transmitter 200 and the needle assembly 170 to make linear motion in a lengthwise direction of the applicator 100.

To this end, the driving portion 150 includes all or part of a spring 151, a wheel 152, and a locking member 153.

The spring 151 is a power source for providing power to the needle carrier 160 so that the needle carrier 160 may make linear motion. To this end, the spring 151 may be a wound reel spring. The spring 151 is compressed and assembled in its initial state. Meanwhile, when an engagement portion for fixing compression of the spring 151 is released by pressing of the button 120, the spring 151 may be tensile to return to its original state. Thus, one end of the spring 151 rotates. That is, this may mean that a type of scotch yoke or double slider crank mechanism in which the rotational motion of the spring 151 is converted into linear motion of the needle carrier 160 and the needle assembly 170, is applied.

One side of the wheel 152 is engaged with one end of the spring 151 and is configured to rotate together by rotation of the spring 151. A groove through which the wheel 152 is to be coupled to the locking member 153, may be formed in an outer circumferential surface of the wheel 152.

The locking member 153 is configured in such a way to have one end to be coupled to the button 120 and the other end of the locking member 153 to be engaged with the wheel 152. In this case, a groove is formed in the other end of the locking member 153 and thus may be engaged with the groove of the wheel 152. Thus, rotation of the wheel 152 is prevented, and furthermore, tension of the spring 151 is prevented.

When the button 120 moves inward toward the applicator 100, the locking member 153 moves in conjunction therewith. As a result, the engagement between the other end of the locking member 153 and the groove of the cap 130 is released, allowing the wheel 152 to rotate together due to the tension and rotation of the spring 151.

The needle carrier 160 is configured to make a linear motion within the upper housing 110 and lower housing 140. In this case, the linear motion of the needle carrier 160 may be induced by the rotational motion of the driving portion 150. To this end, it is preferable that the needle carrier has an overall T-shaped configuration. The needle carrier 160 may be coupled to the other side of the wheel 152 and thus depend on the rotational motion of the driving portion 150.

The needle assembly 170 is disposed in the internal space formed by the upper housing 110 and the lower housing 140. The needle assembly 170 is coupled to the lower end of the needle carrier 160 and configured to make linear motion together, depending on the linear motion of the needle carrier 160.

The needle assembly 170 may include all or part of a needle 171, a grip portion 172, and a needle injection portion 173.

The needle 171 is configured to penetrate the user's skin.

The grip portion 172 is configured to be coupled to the lower end of the needle carrier 160. The needle 171 and the grip portion 172 may be integrally formed, and preferably, they are made entirely of metal, but are not limited thereto.

The needle injection portion 173 is arranged between the needle 171 and the grip portion 172. The needle injection portion 173 is made of a material different from that of the needle 171 and the grip portion 172, preferably rubber. To this end, it is preferable that the needle injection portion 173 is formed by double injection during the manufacturing of the needle assembly 170.

Meanwhile, during the manufacturing of the insertion device assembly, the needle injection portion 173 is inserted into the transmitter 200. Since the needle injection portion 173 is made of rubber, a sealed structure may be formed with the transmitter 200.

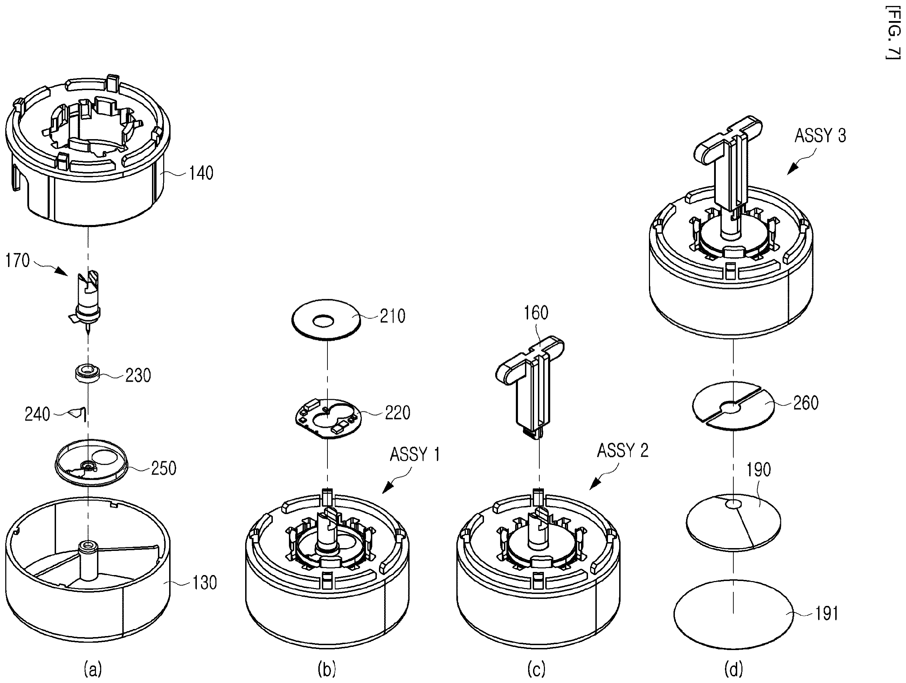

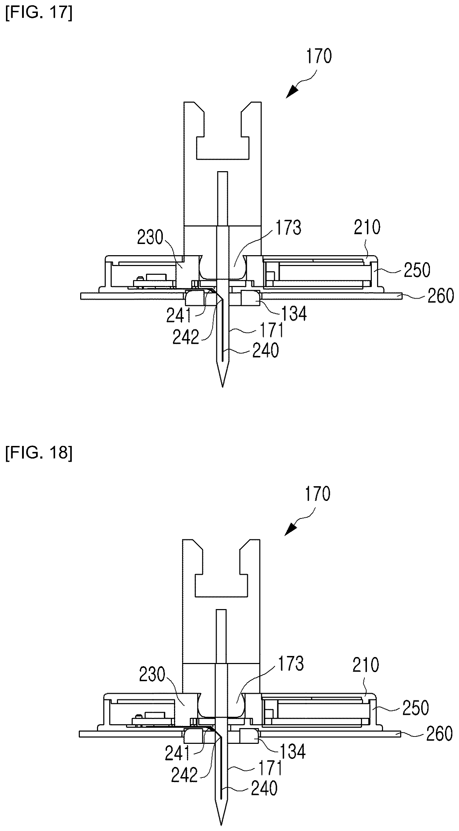

In addition, an end of the needle injection portion 173 protrudes so that the needle injection portion 173 may be inserted from the upper part of the holder 230 of the transmitter 200 (see FIG. 17). Related details are described in FIGS. 7 through 8.

In the needle assembly 170 according to an embodiment of the present disclosure, when the needle assembly 170 is coupled to the transmitter 200 via the needle injection portion 173, rotation may be prevented.

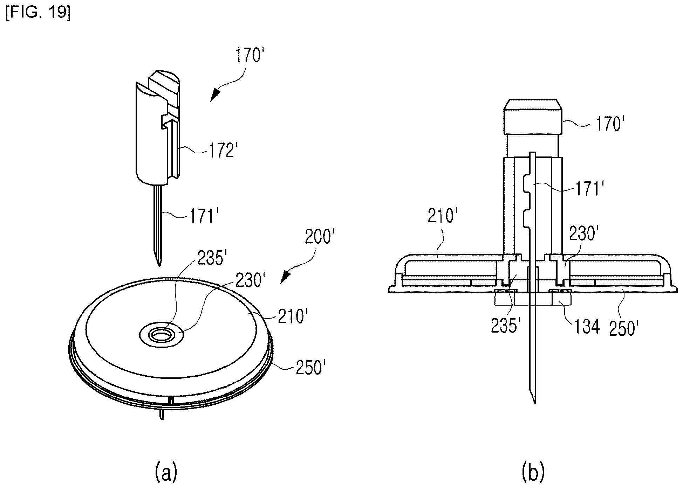

Meanwhile, according to another embodiment of the present disclosure, instead of providing a separate needle injection portion in a needle assembly 170', at least part of the upper surface of a transmitter 200' is formed as an elastic portion, and when a needle 171' is coupled to an upper case 210' of the transmitter 200', a sealed structure is formed while preventing the rotation of the needle 171' (see FIG. 19). Details are described below with reference to FIG. 19.

The transmitter holder 180 is disposed in the internal space formed by the upper housing 110 and the lower housing 140. The transmitter holder 180 is coupled to the transmitter 200 and configured to move the transmitter 200 linearly from distal to proximal. The transmitter holder 180 is pressed from distal to proximal by the needle carrier 160. Meanwhile, the transmitter holder 180 may not move from proximal to distal and may only move from distal to proximal.

The dehumidifier 190 is inserted into the interior of the cap 130 through the bottom surface of the cap 130 and configured to dehumidify the interior of the applicator 100.

The sealing portion 191 is attached to the open bottom surface of the cap 130 and configured to shield the interior and exterior of the applicator 100.

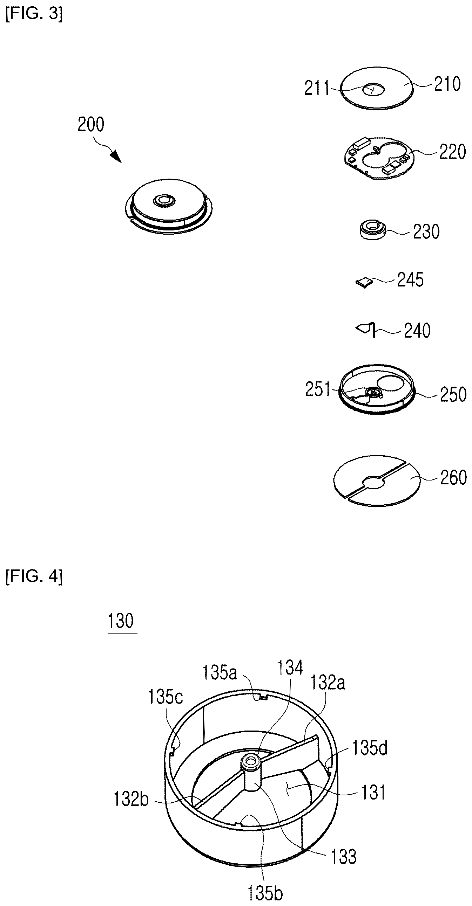

FIG. 3 is a perspective view and exploded perspective view of a transmitter according to an embodiment of the present disclosure.

The transmitter 200 according to an embodiment of the present disclosure is attached to the user's skin and configured to analyze the concentration of analytes in the user's body and transmit concentration-related information to an external device. The transmitter 200 may be inserted into the interior of the applicator 100 during the production stage of the insertion device assembly.

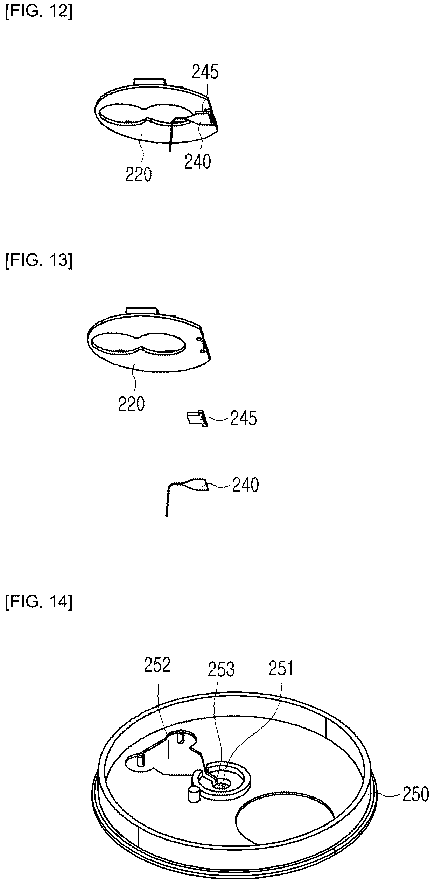

Referring to FIG. 3, the transmitter 200 may include all or part of the upper case 210, a printed circuit board (PCB) 220, a holder 230, a sensor 240, a dummy PCB 245, a lower case 250, and an adhesive tape 260. Details are described below with reference to FIGS. 12 through 17.

FIG. 4 shows the interior of the cap according to an embodiment of the present disclosure.

Referring to FIG. 4, the cap 130 according to an embodiment of the present disclosure may be made of one or more materials selected from acrylonitrile butadiene styrene (ABS), polycarbonate (PC), and polyethylene (PE), or any material commonly used for medical devices like the applicator. For example, PC resin and ABS resin may be combined, but this may be appropriately modified by a designer's choice.

The cap 130 includes all or part of an opening 131, ribs 132a and 132b, a needle receiving portion 133, a cap injection portion 134, and coupling protrusions 135a, 135b, 135c, and 135d.

The opening 131 is formed on the bottom surface of the cap 130.

The ribs 132a and 132b extend radially inward from the inner circumferential surface of the cap 130. According to an embodiment, two ribs 132a and 132b are symmetrically formed, but the present disclosure is not limited thereto, and the number and shape of the ribs 132a and 132b may be appropriately changed.

The ribs 132a and 132b are arranged along the diameter of the cap 130 so that the cap 130 may be bisected when viewed from the top or bottom, i.e., having an approximate θ shape.

The needle receiving portion 133 is formed at the center of the cap 130 and configured to accommodate the needle 171. To this end, the needle receiving portion 133 may include a receiving space and may have a cylindrical shape with an open upper surface.

The needle receiving portion 133 is supported by the rib 132a and another rib 132b.

Since the needle receiving portion 133 is integrally formed with the cap 130, when the applicator 100 is used, a needle protection configuration can be removed at once by only removing the cap 130 and thus, providing convenience to the user.

The cap injection portion 134 is formed on the upper surface of the needle receiving portion 133 and contacts the bottom surface of the lower case 250 of the transmitter 200. Meanwhile, the cap injection portion 134 may be formed by double injection on top of the needle receiving portion 133 during the manufacturing of the cap 130, but the present disclosure is not limited thereto.

Meanwhile, during manufacturing of the insertion device assembly, when the cap injection portion 134 contacts the bottom surface of the lower case 250, and since the cap injection portion 134 is made of rubber, a sealed structure may be formed with the transmitter 200. Related details are described in FIGS. 7 through 8.

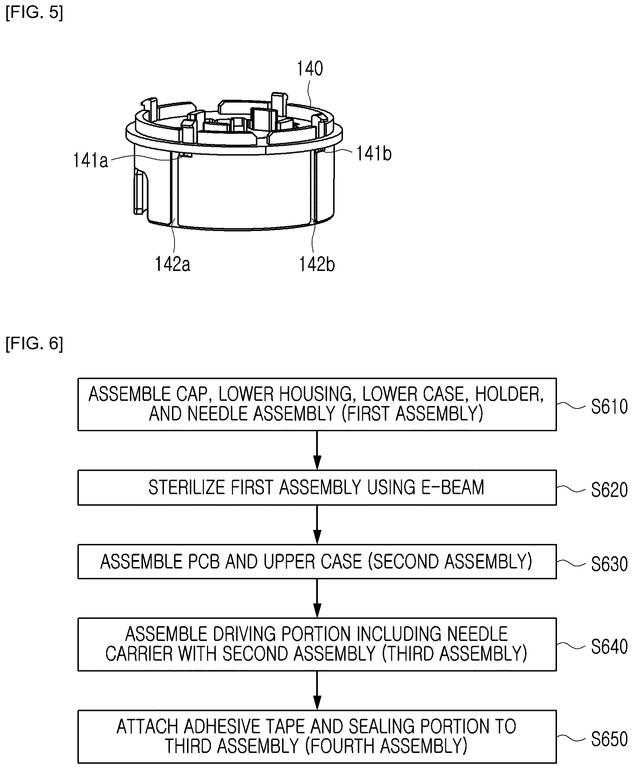

One or more coupling protrusions 135a, 135b, 135c, and 135d protrude radially inward from the inner circumferential surface of the cap 130. One or more coupling protrusions 135a, 135b, 135c, and 135d are configured to be coupled to the outer circumferential surface of the lower housing 140. Related details are described with reference to FIG. 5.

FIG. 5 is a front perspective view of the lower housing 140 according to an embodiment of the present disclosure.

Referring to FIG. 5, the lower housing 140 according to an embodiment of the present disclosure may include one or more coupling grooves 141a and 141b formed on an outer circumferential surface thereof.

The coupling grooves 141a and 141b are recessed radially inward from the outer circumferential surface of the lower housing 140 and configured to receive and engage the coupling protrusions 135a, 135b, 135c, and 135d. In this case, the coupling grooves 141a and 141b are formed with the same number as the number of the coupling protrusions 135a, 135b, 135c, and 135d. Meanwhile, in the present disclosure, although two coupling grooves 141a and 141b are illustrated, but additional coupling grooves may be formed on the opposite side.

Guide grooves 142a and 142b may be further formed on one side of the coupling grooves 141a and 141b. The guide grooves 142a and 142b are recessed radially inward and extend along the height direction of the lower housing 140. Meanwhile, the width of the coupling grooves 141a and 141b may be greater than the width of the coupling protrusions 135a, 135b, 135c, and 135d. Thus, the coupling protrusions 135a, 135b, 135c, and 135d may move vertically from the inside of the coupling grooves 141a and 141b.

In an example, the guide grooves 142a and 142b may be continuously formed to the left of the coupling grooves 141a and 141b. In this case, the coupling protrusions 135a, 135b, 135c, and 135d are coupled to the coupling grooves 141a and 141b and then move to the left along the width of the coupling grooves 141a and 141b and the width of the guide grooves 142a and 142b and then move downward along the height direction of the guide grooves 142a and 142b and may be removed outside the lower housing 140. That is, the user rotates the cap 130 to the right along the outer circumferential surface of the lower housing 140 and then moves the cap 130 downward along the height direction of the lower housing 140 so that coupling between the cap 130 and the lower housing 140 may be released.

Meanwhile, according to an embodiment of the present disclosure, it has been described that the lower housing 140 and the cap 130 may be coupled or uncoupled to each other through the coupling protrusions 135a, 135b, 135c, and 135c and the coupling grooves 141a and 141b, but the present disclosure is not limited thereto. For example, the lower housing 140 and the cap 130 may be coupled to each other using a method such as hook engagement or screw fastening or the like. Alternatively, both may be engaged with each other by an opening method of a wing member (not shown) provided at one of the cap 130 and the lower housing 140 and a catch (not shown) provided at the other one of the cap 130 and the lower housing 130.

### 1.2. Assembly method of applicator

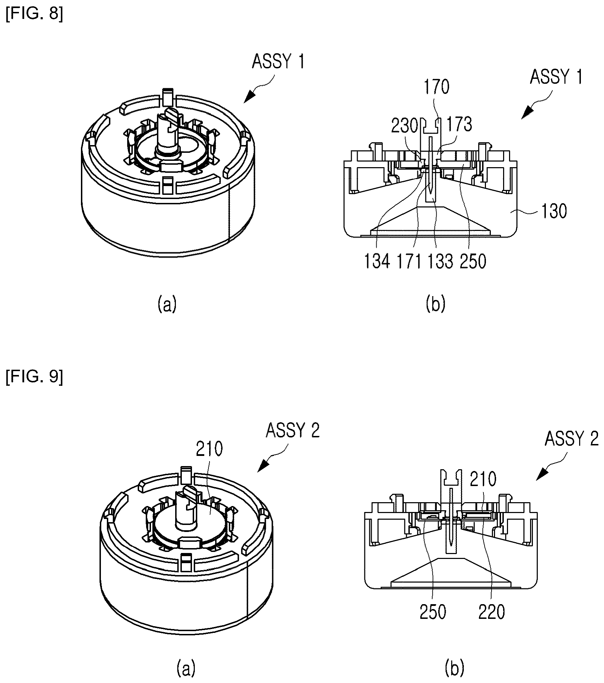

FIG. 6 is a flowchart illustrating an assembly method of the insertion device assembly according to an embodiment of the present disclosure. FIG. 7 illustrates an assembly order of the insertion device assembly according to an embodiment of the present disclosure. FIGS. 8 through 11 illustrate a state of an assembly operation of the insertion device assembly according to an embodiment of the present disclosure.

A method of assembling and sterilizing the insertion device assembly according to an embodiment of the present disclosure will be described with reference to FIGS. 6 through 11.

First, the cap 130, the lower case 250, the sensor 240, the holder 230, the needle assembly 170, and the lower housing 140 are sequentially assembled (S610, (a) of FIG. 7). Hereinafter, an assembly of the cap 130, the lower case 250, the sensor 240, the holder 230, the needle assembly 170, and the lower housing 140 is referred to as a first assembly ASSY 1.

The first assembly ASSY 1 is shown in FIG. 8. (a) of FIG. 8 is a front perspective view of the first assembly ASSY 1, and (b) of FIG. 8 is a cross-sectional view of the first assembly ASSY 1. Referring to (b) of FIG. 8, a protrusion of the needle injection portion 173 is inserted into the holder 230, and frictional coupling may be used as a combination of both, but the present invention is not limited thereto, and another type of coupling may also be used.

When the needle assembly 170 is coupled to the upper part of the transmitter 200, the protrusion of the needle injection portion 173 is inserted into the holder 230 so that contaminants may be prevented from flowing into the needle 171 through the needle injection portion 173 and the holder 230. In addition, by combination of the needle injection portion 173 and the holder 230, sterile conditions may be maintained, and sealing may be performed without using additional parts.

Meanwhile, after the applicator 100 is fired by the user, when the needle assembly 170 retreats, the transmitter 200 stays in a distal end. Thus, the protruding part of the needle injection portion 173 inserted into the holder 230 is withdrawn from the holder 230.

In addition, when the cap injection portion 134 comes into contact with the bottom surface of the lower case 250, a structure for sealing a lower through-hole 251 of the lower case 250 is formed. In this case, due to combination of the cap 130 and the lower housing 140, the cap injection portion 134 may be maintained while coming into contact with the bottom surface of the lower case 250.

At least part of the cap 130 may be formed of a transparent material. Thus, it may be confirmed with the naked eye whether sealing is formed between the cap injection portion 134 and the transmitter 200, so that it is easy to inspect for any defective or unsealed products.

According to the above description, in the insertion device assembly according to an embodiment of the present disclosure, when assembling, in a state of the first assembly ASSY 1, the protrusion of the needle injection portion 173 is inserted into the holder 230, the cap injection portion 134 comes into contact with the bottom surface of the lower case 250, and a double sealing structure for sealing the lower through-hole 251 of the lower case 250 is formed (see FIG. 17) so that contaminants may be prevented from flowing into the needle 171 through the upper side of the transmitter 200, i.e., between the needle injection portion 173 and the holder 230 and contaminants may be prevented from flowing into the needle 171 through the lower side of the transmitter 200, i.e., the lower through-hole 251 and the inside of the insertion device assembly may be protected from contaminants.

In addition, since the needle 171 is a part that penetrates the user's skin directly, it is very significant to maintain sterile conditions, and the double sealing structure of the insertion device assembly according to the present disclosure is quite effective in maintaining sterile conditions.

In addition, the double sealing structure according to an embodiment of the present disclosure may be manufactured without combination of additional parts for sealing and sterilization so that a simple structure for sealing and sterilization may be provided.

Subsequently, E-beam is irradiated onto the first assembly ASSY 1 so that the first assembly ASSY 1 is sterilized (S620). Since an electronic device is mounted on the PCB 220, when the PCB 220 is directly exposed to E-beam, there is a risk of loss of some functions of the electronic device. Meanwhile, according to the present disclosure, since sterilization by E-beam is performed in the state of the first assembly ASSY 1 in which the PCB 220 is not assembled, there is a risk of malfunction of electronic devices mounted on the PCB 220.

The PCB 220, the upper case 210, and the adhesive tape 260 are assembled onto the first assembly ASSY 1 (S630, (b) of FIG. 7). Hereinafter, an assembly to be formed in operation S630 is referred to as a second assembly ASSY 2.

The second assembly ASSY 2 is shown in FIG. 9. (a) of FIG. 9 is a front perspective view of the second assembly ASSY 2, and (b) of FIG. 9 is a cross-sectional view of the second assembly ASSY 2.

Referring to (a) and (b) of FIG. 9, the lower case 210 is coupled to the lower case 250. In this case, the upper case 210 and the lower case 250 may be coupled to each other using one of ultrasonic welding or UV bonding.

Subsequently, the needle carrier 160 and the driving portion 150 are assembled with the second assembly ASSY 2 (S640, (c) of FIG. 7). Meanwhile, in FIGS. 7, 10, and 11, only the needle carrier 160 is coupled to the second assembly ASSY 2, but this is for convenience of explanation, and it will be understood that the driving portion 150 coupled to the needle carrier 160 is assembled with the second assembly ASSY 2 together. Hereinafter, an assembly to be formed in operation S640 is referred to as a third assembly ASSY 3.

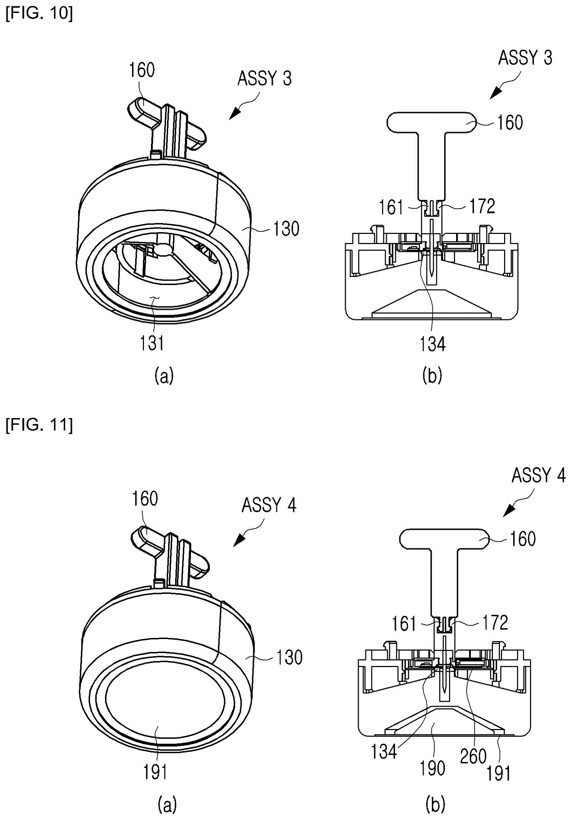

The third assembly ASSY 3 is shown in FIG. 10. (a) of FIG. 10 is a bottom perspective view of the third assembly ASSY 3, and (b) of FIG. 10 is a cross-sectional view of the third assembly ASSY 3.

Referring to (a) of FIG. 10, the opening 131 of the cap 130 is still in an open state.

In addition, referring to (b) of FIG. 10, a coupling arm 161 formed at a lower end of the needle carrier 160 and a grip portion 171 of the needle assembly 170 are engaged with each other so that the needle carrier 160 and the needle assembly 170 may be coupled to each other. In this case, a snap-fit method may be adopted as a combination of the coupling arm 161 and the grip portion 172, but the present disclosure is not limited thereto, and screw fastening or the like instead may be adopted.

Subsequently, the adhesive tap 260, the dehumidifier 190, and the sealing portion 191 are sequentially coupled to the third assembly ASSY 3 (S650, (d) of FIG. 7). Hereinafter, an assembly to be formed in operation S650 is referred to as a fourth assembly ASSY 4.

The fourth assembly ASSY 4 is shown in FIG. 11. (a) of FIG. 11 is a bottom perspective view of the fourth assembly ASSY 4, and (b) of FIG. 11 is a cross-sectional view of the fourth assembly ASSY 4.

Referring to (b) of FIG. 11, the adhesive tape 260 is attached to the bottom surface of the lower case 250. In this case, as described above, the adhesive tape 260 is divided into two pieces and may be inserted through the right and left openings 131 of the cap 130, respectively.

When the adhesive tape 260 is attached, the dehumidifier 190 is inserted into the cap 130, and the cap 130 is sealed by the sealing portion 191.

Meanwhile, according to an embodiment of the present disclosure, in the insertion device assembly, when assembling, only the sealing portion 191 has to be attached to the lower part of the cap 130, so that a sealing method is easy.

Meanwhile, in the present disclosure, after the fourth assembly ASSY 4 is assembled, the upper housing 110 having the button 120 provided thereon is further assembled, but in the present disclosure, related description or detailed drawings are omitted.

### 1.3 Sterilization test result of applicator and transmitter

As sterilization results of an insertion device assembly according to an embodiment or another embodiment of the present disclosure, it was confirmed that no microbial growth was observed.

Specifically, a testing method has been performed according to ISO 11737-2:2019, Sterilization of health care products - Microbiological methods-Part 2: Tests of sterility performed in the definition, validation and maintenance of a sterilization process.

In addition, instruments and equipment used were incubators, low-temperature incubators, high-pressure steam sterilizers, and clean benches.

In addition, cultures in soybean casein digest medium (TSB) were incubated for 28 days (14 days at 30-35°C and 14 days at 20-25°C).

In this case, after culturing microorganisms at E-beam doses of 25 kGy and 35 kGy, respectively, it was confirmed whether microbial growth was observed.

As testing results using the above-described experiment method, in all cases where E-beam doses of 25 kGy and 35 kGy were used, it was confirmed that no microorganisms were grown. That is, it is confirmed that, when the assembling and sterilization method of the insertion device assembly according to embodiments of the present disclosure is adopted, the effect of sterilization and contamination prevention from external contaminants is very excellent.

### 2. Explanation of transmitter

Hereinafter, the configuration of the transmitter 200 of the insertion device assembly according to an embodiment of the present disclosure will be described in detail with reference to FIG. 3 and FIGS. 12 through 17 attached.

As described above, FIG. 3 is a perspective view and exploded perspective view of the transmitter 200 according to an embodiment of the present disclosure.

Referring to FIG. 3, the transmitter 200 may include all or part of an upper case 210, a PCB 220, a holder 230, a sensor 240, a dummy PCB 245, a lower case 250, and an adhesive tap 260.

The upper case 210 and the lower case 250 are vertically coupled to each other and constitute the exterior of the transmitter 200 so that a certain accommodation space may be formed in the transmitter 200.

An upper through-hole 211 is formed in the upper case 210, and a lower through-hole 251 is formed in the lower case 250. According to an embodiment of the present invention, the upper through-hole 211 is formed in the center of the upper case 210, and the lower through-hole 251 is formed in the center of the lower case 250, but the present invention is not limited thereto.

The holder 230 is positioned between the upper through-hole 211 and the lower through-hole 251. The holder 230 may have a cavity shape, but the present invention is not limited thereto. In the state where the needle injection portion 173 is pressed into the holder 230, the needle 171 may be positioned to penetrate the upper case 210 and the lower case 250.

The PCB 220 is a printed circuit board, and is accommodated in an internal accommodation space of the upper case 210 and the lower case 250. Each electronic part for analyzing the concentration of the user's body analytes detected by the sensor 240 and for transmitting information relating to the concentration to an external device is mounted on the PCB 220.

The sensor 240 is positioned at the lower part of the holder 230 in the accommodation space inside the transmitter 200.

The sensor 240 has one side that extends to the outside of the transmitter 200 through the lower through-hole 251 of the transmitter 200 and is disposed within the needle 171 and inserted into the user's body by firing and the other side that is connected to the lower part of the PCB 220 inside the transmitter 200.

Hereinafter, one end of the sensor 240 that is inserted into the user's body outside the transmitter 200 may be referred to as an insertion portion, and the other end of the sensor 240 that is connected to the PCB 220 inside the transmitter 200 may be referred to as a connection portion.

The dummy PCB 245 will be described with reference to FIGS. 12 and 13. FIGS. 12 and 13 are a perspective view and an exploded perspective view of a connecting structure of the PCB 220, the sensor 240, and the dummy PCB 245 as viewed from below.

The dummy PCB 245 is positioned between the sensor 240 and the PCB 220.Likewise, the dummy PCB 245 includes a small printed circuit board and electrically connects the electronic parts mounted on the sensor 240 and the PCB 220.

Unlike the PCB 220, parts or the like are not mounted on the dummy PCB 245, and only a printed circuit for electrically connecting the sensor 240 and the PCB 220 is included.

When specifically describing with reference to FIGS. 12 and 13, the dummy PCB 245 is positioned at the lower part of the PCB 220 and the upper part of the sensor 240, and one side of the dummy PCB 245 contacts a connection part of the sensor 240, and the other side of the dummy PCB 245 contacts a contact point of the circumference of the PCB 220, so that the sensor 240 and the PCB 220 may be physically and electrically connected to each other.

The sensor 240 and the dummy PCB 245 are connected by ultrasonic welding or anisotropic conductive film (ACF) bonding, and the dummy PCB 245 and the PCB 220 may be connected by soldering.

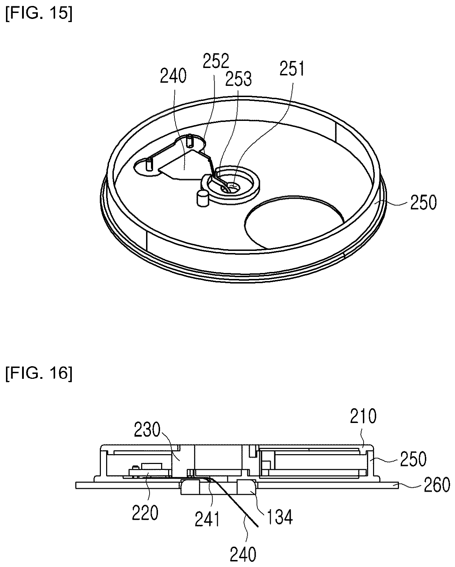

FIGS. 14 and 15 are a perspective view of the lower case 250 and illustrates a track structure positioned at an inner surface of the lower case 250 and a state in which the sensor 240 is installed at the lower case 250.

Referring to FIG. 14, a fixed track 252 is positioned at an upper surface of the lower case 250.

The fixed track 252 is formed in a shape in which one side of the lower case 250 is concavely stepped to correspond to the connection portion of the sensor 240, and the connection portion of the sensor 240 is fitted into the fixed track 252, so that the sensor 240 may be fixed on the lower case 250.

An inclined track 253 is positioned at one side of the fixed track 252. The inclined track 253 is formed in a shape in which the lower through-hole 251 of the fixed track 252 is inclined downward.

Referring to FIG. 15, when the sensor 240 is fitted into the fixed track 252, a part between the connection portion and the insertion portion of the sensor 240 is disposed on the inclined track 253.

FIG. 16 is a longitudinal cross-sectional view of the shape of the sensor 240 in a state in which the needle assembly 170 is not coupled to the transmitter 200, and FIG. 17 is a longitudinal cross-sectional view of the sensor 240 in a state in which the needle assembly 170 is coupled to the transmitter 200.

Referring to FIGS. 16 and 17, a part between the connection portion and the insertion portion of the sensor 240 is disposed on the inclined track 253, so that the sensor 240 that extends to the outside of the lower housing through the lower through-hole 251 constitutes a first bent portion 241 that is bent while forming an obtuse angle centering on the inclined track 253, and in this state, when the needle assembly 170 is coupled to the sensor 240, one end of the sensor 240, i.e., the insertion portion is accommodated in the needle 171, and the sensor 240 constitutes a second bent portion 242 that is bent while forming an obtuse angle. That is, the middle part of the sensor 240 is not directly bent at a right angle, but two bent portions that form an obtuse angle are formed between one end and the other end of the sensor 240, and one end and the other end of the sensor 240 form a right angle with the two bent portions therebetween.

Meanwhile, in the present disclosure, for convenience of explanation, the bent portions 241 and 242 are formed by a discontinuous surface, but it should be noted that the bent portions 241 and 242 may have a continuously bent shape.

Since the sensor 240 is manufactured in a flexible film, it may be bent; however, when the sensor 240 is folded while being bent at a small angle, material sputtered onto an electrode of the sensor 240 or the sensor 240 may be damaged by cracks, and in this way, two bent portions are formed on the sensor 240 so that damage of a film that constitutes the sensor 240 and material sputtered on the film may be prevented.

In addition, the sensor 240 forms a certain angle downward by the first bent portion 241 before the needle 171 is assembled with the sensor 240, so that assembling of the needle 171 coupled to the upper part of the sensor 240 through the lower part from the upper part of the transmitter 200 may be easily performed.

Tension applied to the electrode in a state in which the sensor is inserted into and fixed on the user's skin may be reduced.

The adhesive tape 260 is positioned on the bottom surface of the lower case 250, and the transmitter 200 is adhered to the user's skin and fixed thereto.

An opening is formed in the adhesive tape 260 to allow the sensor 240 extending from the lower case 250 to pass through and be positioned.

Referring back to FIG. 3, the adhesive tape 260 is configured to be divided depending on the position and the number of ribs 132a and 132b formed inside the cap of the applicator.

In the present embodiment, two ribs 132a and 132b are arranged along the diameter of the cap 130, so that, when the cap 130 is viewed from the bottom or top, the cap 130 is configured in an approximately θ shape in a bisected shape and thus, the adhesive tape 260 is configured on the bottom surface of the lower case 250 in a bisected θ shape in which the center is divided into two halves.

Thus, in a state in which the adhesive tape 260 divided depending on the shape of the ribs 132a and 132b is attached to the bottom surface of the lower case 250, the adhesive tap 260 may be inserted through the right and left openings 131 of the cap 130, respectively.

In addition, as shown in FIG. 18, in the transmitter 200 according to an embodiment of the present disclosure, the holder 230 may be formed integrally with the upper case 210.

Specifically, by injection molding the holder 230 to allow the upper part of the holder 230 to extend from the upper inner side of the upper case 210 downward, the holder 230 may be formed integrally with the upper case 210.

Thus, the assembly of the transmitter 200 may be easily performed, and simultaneously, the upper part of the transmitter 200 may be more securely sealed.

FIG. 19 illustrates coupling between a needle assembly and a transmitter according to another embodiment of the present disclosure.

(a) of FIG. 19 is a front perspective view of a state before the needle assembly 170' is coupled to the transmitter 200', and (b) of FIG. 19 is a cross-sectional view of a state in which the needle assembly 170' is coupled to the transmitter 200'.

Referring to (a) of FIG. 19, the needle assembly 170' according to another embodiment includes the needle 171' and the grip portion 172'. In addition, the transmitter 200' according to another embodiment may further include a packing portion 235'.

The packing portion 235' is positioned inside the holder 230' positioned in the upper case 210' and the lower case 250'. According to an embodiment of the present invention, the holder 230' may have a cavity shape, and the packing portion 235' may have a shape corresponding thereto.

The packing portion 235' is formed of a rubber material having elasticity and seals the opening of the upper part of the holder 230.

FIG. 19 illustrates that the packing portion 235' as an additional configuration is positioned at the upper part of the holder 230', but the packing portion 235' may be manufactured integrally with the holder 230' by double injection.

As described above, the sterilization and assembly process is performed with the needle 171' of the needle assembly 170' penetrating the packing portion 235', and after the applicator is fired by the user, the needle assembly 170' is retracted, the needle 171' inserted into the holder 230' is removed from the holder 230', and a part that the needle 171' penetrates is closed by the elasticity of the rubber. Accordingly, when the transmitter 200' is attached and used, a part that the needle 171' penetrates is closed by the packing portion 235' and is not exposed to the outside, and in particular, foreign substances such as water may be prevented from entering due to hydrophobicity of the rubber material so that infection caused thereby may be reduced.

In the present embodiment as well, in the state of the first assembly ASSY 1 described above, the cap injection portion 134 comes into contact with the bottom surface of the lower case 250' and seals the lower through-hole 251 of the lower case 250, thereby forming the double sealing structure described above.

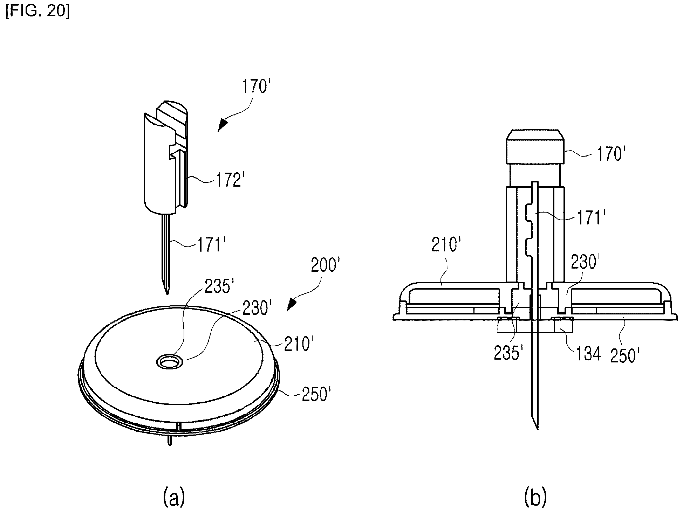

In addition, as shown in FIG. 20, in the transmitter 200' according to the present embodiment, the holder 230' is formed integrally with the upper case 210', so that the same effect as in the above embodiment may be attained.

While the present invention has been particularly shown and described with reference to exemplary embodiments thereof, it will be understood by those of ordinary skill in the art that various changes in form and details may be made therein without departing from the spirit and scope of the present invention as defined by the following claims.

### (Explanation of reference numerals)

100: applicator
110: upper housing
120: button
130: cap
140: lower housing
150: driving portion
160: needle carrier
170, 170': needle assembly
180: transmitter holder
190: dehumidifier
191: sealing portion
200, 200': transmitter
210, 210': upper case
220: PCB
230, 230': holder
235': packing portion
240: sensor
245: dummy PCB
250,250': lower case
252: fixed track
253: inclined track
260: adhesive tape

## Claims

1. An insertion device assembly comprising:
a transmitter; and
wherein the transmitter comprises:
an upper case;
a lower case coupled to the upper case and forming an accommodation space therein;
a sensor arranged on the lower case;
a holder arranged inside the lower case and arranged on the sensor; and
a lower through-hole, which is positioned in the lower case and in which the holder is positioned;
an applicator on which the transmitter is mounted,
wherein the applicator comprises:
an upper housing having an opening formed in a lower part of the upper housing and a button provided on an outer circumferential surface of the upper housing;
a lower housing coupled to the opening of the upper housing, thereby forming an internal space with the upper housing;
a needle assembly arranged at an upper part of the transmitter in the internal space and configured so that at least part of the needle assembly is inserted into the holder;
a needle carrier arranged in the internal space and configured to allow the needle assembly to make linear motion; and
a driving portion configured to provide power to the needle carrier,
wherein the needle assembly comprises:
a needle configured to penetrate a user's skin;
a grip portion coupled to one end of the needle carrier;
a needle injection portion arranged between the needle and the grip portion and configured to be coupled to the holder; and
a protrusion protruding from an end of the needle injection portion and inserted into the holder.

2. An insertion device assembly comprising:
a transmitter; and
wherein the transmitter comprises:
an upper case;
a lower case coupled to the upper case and forming an accommodation space therein;
a sensor arranged on the lower case;
a holder arranged inside the lower case and arranged on the sensor; and
a lower through-hole, which is positioned in the lower case and in which the holder is positioned; and
a packing portion positioned at the upper part of the holder and formed of an elastic material,
an applicator on which the transmitter is mounted,
wherein the applicator comprises:
an upper housing having an opening formed in a lower part of the upper housing and a button provided on an outer circumferential surface of the upper housing;
a lower housing coupled to the opening of the upper housing, thereby forming an internal space with the upper housing;
a needle assembly arranged at an upper part of the transmitter in the internal space and configured so that at least part of the needle assembly is inserted into the holder;
a needle carrier arranged in the internal space and configured to allow the needle assembly to make linear motion; and
a driving portion configured to provide power to the needle carrier, wherein the needle assembly comprises:
a needle configured to penetrate a user's skin;
a grip portion coupled to one end of the needle carrier; and
a needle injection portion arranged between the needle and the grip portion and configured to be coupled to the holder.

3. The insertion device assembly of claim 1, wherein, when the insertion
device assembly is assembled, the protrusion is inserted into the holder, and when the insertion device assembly is used, the needle injection portion is withdrawn from the holder.

4. The insertion device assembly of claim 2, wherein, when the insertion
device assembly is assembled, the needle is positioned to penetrate the packing portion, and when the insertion device assembly is used, the needle is removed from the holder, and a part of the packing portion that the needle penetrates, is closed by elasticity.

5. The insertion device assembly of claim 1 or 2, wherein the upper part of the
holder and an inner upper surface of the upper case are integrally injection molded.

6. The insertion device assembly of claim 1 or 2, further comprising a cap
configured to be mounted on or removed from the upper housing,
wherein the cap comprises:
an opening formed in a lower surface of the cap;
a plurality of ribs of which at least part is arranged inside the lower housing and which extend radially inward from an inner circumferential surface of the cap; and
a needle accommodation portion supported by the plurality of ribs and configured to surround at least part of the needle.

7. The insertion device assembly of claim 6, wherein the needle assembly
further comprises a cap injection portion that corresponds to a shape of an upper surface of the needle injection portion and arranged on an upper surface of the needle accommodation portion, and the cap injection portion comes into contact with the lower through-hole of the lower case and seals the lower through-hole of the lower case.

8. The insertion device assembly of claim 7, wherein the cap injection portion includes an elastic member.

9. The insertion device assembly of claim 6, wherein the plurality of ribs are
two or more and are arranged symmetrically around the needle accommodation portion, and the transmitter further comprises an adhesive tape attached to a lower surface of the lower case and provided with a number of the plurality of ribs, and the adhesive tape is capable of being inserted through the opening.

10. The insertion device assembly of claim 1 or 2, wherein the transmitter
further comprises a printed circuit board (PCB) accommodated in an accommodation space of the upper case and the lower case and a dummy PCB connecting the sensor to the PCB.

11. The insertion device assembly of claim 1 or 2, wherein the transmitter
further comprises an inclined surface formed to be inclined from one side of the lower through-hole downward, and the inclined surface further comprises an inclined track on which part of the sensor is disposed.

12. The insertion device assembly of claim 11, wherein the insertion portion of
the sensor is arranged inside the needle that penetrates the holder and is positioned, and two bent portions that form an obtuse angle are formed between one end and the other end of the sensor by the inclined surface and the needle.

13. An insertion device assembly comprising:
a transmitter; and
an applicator configured in such a way that the transmitter is mounted on the applicator,
wherein the insertion device assembly is divided into first through fourth assemblies depending on an assembly order, and
the first assembly comprises:
a cap of which upper and lower parts are open;
a lower housing arranged inside the cap;
a lower case of the transmitter arranged on at least part of the lower housing;
a sensor arranged on the lower case;
a holder arranged inside the lower case and arranged on the sensor; and
a needle assembly arranged at an upper part of the transmitter and configured in such a way that a protruding end of the needle assembly is inserted into the holder.

14. An insertion device assembly comprising:
a transmitter; and
an applicator configured in such a way that the transmitter is mounted on the applicator,
wherein the insertion device assembly is divided into first through fourth assemblies depending on an assembly order, and
the first assembly comprises:
a cap of which upper and lower parts are open;
a lower housing arranged inside the cap;
a lower case of the transmitter arranged on at least part of the lower housing;
a sensor arranged on the lower case;
a holder arranged inside the lower case and arranged on the sensor;
a packing portion positioned at an upper part of the holder and formed of an elastic material; and
a needle assembly, which is arranged at an upper part of the transmitter and in which a needle positioned at an end of the needle assembly is positioned to penetrate the packing portion.

15. The insertion device assembly of claim 13 or 14, wherein, to the first assembly sterilized using E-beam, the second assembly further comprises:
a PCB arranged on the lower case; and
an upper case, which is coupled to the lower case and forms an accommodation space therein.

16. The insertion device assembly of claim 13 or 14, wherein, to the second assembly, the third assembly further comprises:
a needle carrier configured to allow the needle assembly to make linear motion; and
a driving portion configured to provide power to the needle carrier.

17. The insertion device assembly of claim 16, wherein, to the third assembly, the fourth assembly further comprises:
an adhesive tap attached to a lower surface of the lower case; and
a sealing portion configured to seal an open lower part of the cap.

18. The insertion device assembly of claim 13 or 14, wherein the needle assembly comprises:
a needle configured to penetrate a user's skin;
a grip portion coupled to one end of the needle carrier;
a needle injection portion arranged between the needle and the grip portion and configured to be coupled to the holder; and
a protrusion protruding from an end of the needle injection portion and inserted into the holder.

19. The insertion device assembly of claim 13 or 14, wherein the cap
comprises:
a plurality of ribs of which at least part is arranged inside the lower housing and which extend radially inward from an inner circumferential surface of the cap; and
a needle accommodation portion supported by the plurality of ribs and configured to surround at least part of the needle; and
a cap injection portion having a shape corresponding to a shape of an upper surface of the needle accommodation portion and arranged on the upper surface of the needle accommodation portion, and
the cap injection portion is positioned to contact a lower through-hole positioned in a lower surface of the lower case.

20. The insertion device assembly of claim 19, wherein the plurality of ribs are
two or more and are arranged symmetrically around the needle accommodation portion, and the transmitter further comprises an adhesive tape attached to a lower surface of the lower case and provided with a number of the plurality of ribs, and the adhesive tape is capable of being inserted through the open lower part of the cap.
